(19) Europäisches Patentamt
European Patent Office
Office européen des brevets

(11) **EP 1 767 239 B1**

(12) **EUROPEAN PATENT SPECIFICATION**

(45) Date of publication and mention
of the grant of the patent:
**10.12.2008 Bulletin 2008/50**

(51) Int Cl.:
*A61M 25/09* (2006.01)

(21) Application number: **05256018.2**

(22) Date of filing: **27.09.2005**

(54) **A medical guide wire**

Medizinischer Führungsdraht

Fil de guidage médical

(84) Designated Contracting States:
**AT BE BG CH CY CZ DE DK EE ES FI FR GB GR
HU IE IS IT LI LT LU LV MC NL PL PT RO SE SI
SK TR**

(43) Date of publication of application:
**28.03.2007 Bulletin 2007/13**

(60) Divisional application:
**07020121.5 / 1 872 821**

(73) Proprietor: **ASAHI INTECC CO., LTD.
Nagoya-shi,
Aichi-ken (JP)**

(72) Inventor: **Kato, Tomihisa
Nagoya-shi Aichi-ken (JP)**

(74) Representative: **Price, Nigel John King et al
J.A. Kemp & Co.
14 South Square
Gray's Inn
London WC1R 5JJ (GB)**

(56) References cited:

| | |
|---|---|
| **EP-A- 0 666 086** | **EP-A- 0 803 266** |
| **EP-A- 0 982 046** | **EP-A- 1 243 283** |
| **EP-A- 1 520 600** | **EP-A1- 1 498 152** |
| **EP-A1- 1 576 980** | **WO-A-99/44665** |
| **JP-A- 2005 278 795** | **US-A- 4 759 463** |
| **US-A- 5 217 026** | **US-A- 5 443 907** |
| **US-A1- 2004 167 436** | **US-B1- 6 390 992** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to a medical guide wire and a method of making the medical guide wire which is improved at manipulatability so that it can navigate deeply into the vascular system with an efficient use of the blood streams.

Upon implementing a therapeutical treatment, prior to using a catheter, it is necessary to firstly insert a medical guide wire (referred merely to as "guide wire" hereinafter) into a blood vessel as a guide for the catheter. In order to make the guide wire reachable at desired areas of sinuously curved portions in the vascular system, various contrivances have been suggested.

By way of illustration, Japanese Laid-open Patent Application No. 2000-135289 discloses a guide wire in which a radiopaque helical spring wire is connected to a distal end of an elongation core with the helical spring wire coated by a synthetic tube. On an outer surface of the helical spring wire, a hydrophilic polymer is coated to insure a smoothness and slidability so as to protect the helical spring wire against the thrombi formation. The distal end portion of the elongation core is thinned to insure a good pushability upon inserting it into the blood vessel.

Japanese Laid-open Patent Application JP 4009162 discloses a guide wire, a distal end portion of which is highly flexible with a main portion maintained highly rigid. Into the distal end portion of the guide wire, an X-ray opaque metal is embedded with the distal end portion coated by a synthetic layer. The synthetic layer exhibits a lubricity to insure a good pushability and retractability upon manipulating the guide wire.

Japanese Utility Model Registration No. 2588582 discloses a guide wire in which an elongation core has a distal end portion connected to a radiopaque helical spring. The elongation core is covered by a synthetic coat and a hydrophilic polymer to insure a good manipulatability with a friction reduced against the blood vessel.

EP1498 152 A1 and EP0982046A1 disclose guide wires with chambers in their distal tips. EP0666086A1 discloses a method of making a guide wire coil. With these related arts in mind, none of the guide wires has an intention to make an effective use of a buoyance developed in the blood streams in order to insure a good manipulatability upon navigating the guide wire through the blood vessel.

Therefore, it is an object of the invention to make an effective use of a buoyance developed in the blood streams, and provide a medical guide wire which forms a flotage chamber to improve a manipulatability upon steering the guide wire even when the guide wire has a distal end portion liable to hang in the blood streams under the influence of the gravity.

## SUMMARY OF THE INVENTION

According to the invention, there is provided a medical guide wire as specified in claim 1.

With the flotage chamber formed in the radiopaque portion of the helical spring, a buoyance developed in the blood streams helps prevent the distal end portion of the guide wire from hanging in the blood streams even when the guide wire has the distal end portion liable to hang under the influence of the gravity.

Preferably the thickness of the distal end portion stepwisely decreases as approaching said distal end of said elongate core so as to form a multi-stepped flat portion at a distal end portion of said elongate core.

With the multi-stepped flat portion provided at a distal end portion of the elongation core, it is possible to insure a larger spatial area as the flotage chamber between the elongation core and the helical spring in comparison to an analogous guide wire in which an elongation core is formed into a tapered configuration.

Due to the multi-stepped flat portion, it is possible to bend stepped sections of the multi-stepped flat portion at different radii of curvature. This enables a manipulator to readily bend the distal end portion of the elongation core and insert it deep into a stenotic area staunchly along a sinuously formed path.

Preferably the helical spring is provided by a radiopaque wire and a radiotransparent wire connected by means of a welding procedure, and extruded to be diametrically reduced so as to define a radiopaque helical spring at the distal end portion of the helical spring.

Upon connecting radiopaque helical spring and a radiotransparent helical spring, the former is usually screwed into the latter, and these two springs are fixed at a screwed portion by means of brazing procedure or the like in a prior art counterpart.

With the radiopaque wire and the radiotransparent wire connectedly welded prior to forming them into a helically coiled configuration, it is possible to eliminate the necessity of the brazing material or the like, thus contributing to making it lightweight and provide a more buoyance with the guide wire.

Preferably the flotage chamber is hermetically sealed by a brazing material or the like which fixes the elongation core to the helical spring, and the synthetic coat which covers the outer surface of the helical spring.

This effectively prevents a gaseous component from escaping out of the flotage chamber so as to maintain a good buoyant function of the flotage chamber.

According to the invention, the flotage chamber is hermetically sealed by a brazing material or the like which fixes the elongation core to the helical spring, and the synthetic coat which covers the outer surface of the helical spring. The synthetic coat may include a solid layer as a first hydrophobic layer and a fluid layer serving as a second lubricating layer to exhibit a lubricity when moistened.

The solid layer may have a hydrophobic coat formed by polyurethane, polyether block amide, polyethylene, polyamide or fluoric polymer. The fluid layer has a hydrophilic coat formed by polyvinylpyrrolidone, maleic anhydride ethylester copolymer or polyethylene oxide.

The synthetic coat may be a mixture of a hydrophilic polymer and a hydrophobic polymer, and having a first layer which contains the hydrophobic polymer more than the hydrophilic polymer by weight, and having an outer layer which contains the hydrophilic polymer more than the outer layer contains the hydrophilic polymer by weight. The hydrophilic polymer of the outer layer increases progressively by weight as approaching an outer surface of the outer layer.

The hydrophobic polymer used as the mixture of the hydrophilic polymer is represented by cellulose ester, or copolymer of polymethylvynlether and maleic anhydrite. Among these polymers, cellulose ester is the most preferable selection. In order to improve the flexibility of the hydrophobic polymer, a plasticizer such as camphor, castor oil or dioctyl phthalate may be added.

Preferably a foamy body is placed in the flotage chamber formed by means of the brazing material or the like between the helical spring and the elongation core.

With the foamy body encapsulated into the flotage chamber, it is possible to prevent the elongation core and the helical spring from being unfavorably deformed so as to increase an elastic restitution after manipulatively bent.

Alternatively cotton fibers or a bundle of fibers is placed in the flotage chamber formed by means of the brazing material or the like between the helical spring and the elongation core.

With the cotton fibers or the bundle of fibers easily adjustable in its quantity, it is possible to readily form the flotage chamber without hindering the flexibility required for the distal end portion of the guide wire.

Alternatively foamy beads or microballoons are placed in the flotage chamber formed by means of the brazing material or the like between the helical spring and the elongation core.

With the foamy beads or microballoons having less chances to come in contact with the neighboring beads or microballoons, it is possible to insure larger spatial portions favorable to provide the flotage chamber. With the use of inorganic microballoons, it is possible to increase a contractile strength for the distal end portion of the guide wire exhibited when manipulatively bent without leaking a gaseous component out of the flotage chamber.

A proximal end portion of the elongation core is defined by connecting a multi-stranded helical spring tube.

When comparing a solid core to the case in which the solid core is inserted to the multi-stranded helical spring under the common diametrical dimension at the proximal end portion, the multi-stranded helical spring tube develops a concave-shaped clearance between the neighboring helical line elements. This makes it possible to produce the lightweight elongation core as opposed to a solid elongation core.

Upon inserting the elongation core into the blood vessel, the blood streams run along the helical line elements to give the elongation core a propelling force, thus enabling the manipulator to navigate it deep into the stenotic area of the blood vessel.

The distal end portion of an elongation core may be diametrically reduced gradually. The distal end portion of the elongation core is severed by a predetermined length, and the distal end portion is pressed into a multi-stepped flat configuration after severing the distal end portion. A helical spring is inserted into an outer surface of the distal end portion of the elongation core to fix the helical spring to the elongation core. A hydrophilic polymer is applied to a synthetic coat by means of a dipping procedure after forming the synthetic coat entirely over an outer surface of the helical spring.

With the guide wire produced by the above method, it is possible to hermetically seal the flotage chamber and reduce the friction of the helical spring against the vascular wall.

With the multi-stepped flat portion provided at a distal end portion of the elongation core, it is possible to insure a larger spatial area as the flotage chamber between the elongation core and the helical spring.

Due to the multi-stepped flat portion, it is possible to bend stepped sections of the multi-stepped flat portion at different radii of curvature, thus enabling the manipulator to readily bend the distal end portion of the elongation core and insert it deep into a stenotic area staunchly along a sinuously formed path.

Preferably, the synthetic coat is prepared from a mixture of a hydrophilic polymer and a hydrophobic polymer, and applying the synthetic coat to the helical spring by means of a dipping procedure so that a mixing ratio of the hydrophilic polymer progressively increases as approaching an outer surface of the synthetic coat.

The guide wire produced by the above method is such that it is possible to more hermetically seal the flotage chamber and more reduce the friction of the helical spring against the vascular wall.

As aforementioned, the hydrophobic polymer used as the mixture of the hydrophilic polymer is represented by cellulose ester, or copolymer of polymethylvynlether and maleic anhydrite. Among these polymers, cellulose ester is the most preferable selection.

In an embodiment, the distal end portion of the elongation core formed into a multi-stepped flat configuration has a structure that the distal end portion is deformed with its cross section uniformly maintained through its lengthwise direction.

As for the distal end portion of the elongation core which is to be formed into a multi-stepped flat configuration with its latitudinal cross section uniformly maintained through its lengthwise direction, the distal end portion of the elongation core is in an equi-diametrical bar before subjecting to the pressing procedure.

As for a distal end portion of an elongation core which is to be formed into a multi-stepped flat configuration with its latitudinal cross section changed differently through its lengthwise direction, the distal end portion of an elongation core is in a taper-shaped bar before subjecting to the pressing procedure.

Upon pressing the tapered bar by means of a mould die, the pressing procedure produces a rotational moment in a direction to make the tapered bar tilt so as to render the minute dimensions of the distal end portion unstable, while at the same time, reducing the service life of the mould die.

As opposed to the tapered bar, the pressing procedure deforms the equi-diametrical bar steady so as to render the minute dimensions of the distal end portion stable, while at the same time, lengthening the service life of the mould die.

Preferably an outer diameter of the medical guide wire is 0.2541 mm (0.01 inches) and the medical guide wire is adaptd to be inserted into the guiding catheter, an inner diameter of which ranges from 1.7 mm (5F) to 2.0 mm (6F).

With the buoyance used to float the flotage chamber in the blood streams, it is possible to navigate the distal end portion of an elongation core deep into the blood vessel. With the thinned elongation core, it is possible to thin a catheter so as to render it less intrusive against the diseased area, thus mitigating the burden the patient owes.

Fig. 1 is a longitudinal cross sectional view of a medical guide wire according to a first embodiment of the invention;

Fig. 2 is a latitudinal cross sectional view taken along the line I-I of Fig. 1;

Fig. 3 is a plan view of an elongation core;

Fig. 4 is a side elevational view of the elongation core;

Fig. 5 is a schematic view of comparing an equi-diametrical bar to a taper-shaped bar when shaped into a multi-stepped configuration;

Fig. 6 is an explanatory view of the elongation bar, a distal end portion of which is formed into the multi-stepped configuration;

Fig. 7 is an enlarged longitudinal cross sectional view of a medical guide wire according to a second embodiment of the invention;

Fig. 8 is a latitudinal cross sectional view taken along the line III-III of Fig. 7;

Fig. 9 is a latitudinal cross sectional view taken along the line III'-III' of Fig. 7;

Fig. 10 is an enlarged longitudinal cross sectional view of the medical guide wire;

Fig. 11 is a side elevational view of a medical guide wire according to a third embodiment of the invention but partly sectioned;

Fig. 12 is a longitudinal cross sectional view of a main part of a medical guide wire according to a fourth embodiment of the invention;

Fig. 13 is a longitudinal cross sectional view of a main part of a medical guide wire according to a fifth embodiment of the invention;

Fig. 14 is a longitudinal cross sectional view of a main part of a medical guide wire according to a sixth embodiment of the invention;

Fig. 15 is a longitudinal cross sectional view of a main part of a medical guide wire according to a seventh embodiment of the invention; and

Fig. 16 is a longitudinal cross sectional view of a main part of a medical guide wire according to an eighth embodiment of the invention.

In the following description of the depicted embodiments, the same reference numerals are used for features of the same type.

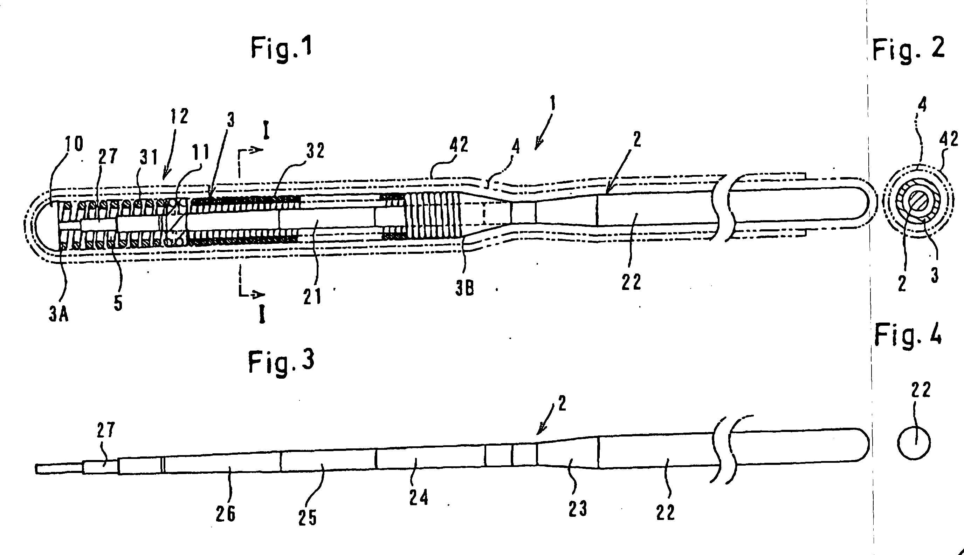

Referring to Figs. 1 through 4 which show a medical guide wire 1 according to a first embodiment of the invention, the medical guide wire 1 (referred only to as "guide wire 1" hereinafter) has an elongation core 2 and a helical Spring 3 inserted to an outer surface of a distal end portion 21 of the elongation core 2. The elongation core 2 is formed by a stainless steel wire, and having the distal end portion 21 extended by approx. 300 mm in length with the rest of the guide wire 1 as a proximal end portion 22 extending by approx. 1200 mm or 2700 mm in length.

The distal end portion 21 has an acutely tapered portion 23, a moderately tapered portion 24, a columnar portion 25, a moderately tapered portion 26 and a multi-stepped flat portion 27 (e.g., 0.04 mm, 0.05 mm and 0.063 mm in thickness from the distal end to the proximal end portion).

In this instance, the distal end portion of the elongation core 2 pressingly formed into a multi-stepped flat configuration has such a structure that the distal end portion 21 is pressed with its latitudinal cross section uniformly maintained through its lengthwise direction. The pressing procedure deform the distal end portion 21 steady so as to render the minute dimensions of the multi-stepped flat portion 27 stable, while at the same time, lengthening the service life of a mould die.

Upon forming the helical spring 3, a platinum wire and a stainless steel wire are prepared which are connectedly welded each other, and extruded to be diametrically reduced before helically wound. The helical spring 3 measures approx. 300 mm, a length of which is substantially the same as the distal end portion 12 of the elongation core 2. The helical spring 3 forms a front helical spring tube 31 and a rear helical spring tube 32. The front helical spring tube 31 serves as a radiopaque portion (approx. 50 mm in length), and the rear helical spring tube 32 serves as a radiotransparent portion (approx. 250 mm in length).

A distal end of the helical spring 3 is air-tightly secured to a distal end of the elongation core 2 by means of a brazing procedure, and a proximal end of the helical spring 3 is air-tightly secured to a proximal end of the elongation core 2 by means of a brazing procedure.

On the outer surface of the helical spring 3 and a proximal end portion 22 of the elongation core 2, are covered by a synthetic coat 4 such as urethane layer or the like. On an outer surface of the synthetic coat 4, is covered by a viscous fluid layer 42 (e.g., polyvinylpyrrolidone selected among the hydrophilic polymer).

At a conjunction between the front helical spring tube 31 and the rear helical spring tube 32, there is provided a hermetical wall 11 by means of a brazing procedure. Within the front helical spring tube 31, a flotage chamber 5 is formed as an inner space surrounded by a brazed portion 10, the hermetical wall 11 and the synthetic coat 4. The structure is such that the flotage chamber 5 is placed at a distal portion 12 of the guide wire 1.

The floatge chamber 5 works as follows:

(a) The platinum metal (21.4 in terms of density relative to water) employed to the front helical spring tube 31 is approx. 2.7 times as heavy as the stainless steel (7,9 in terms of density relative to water).

(b) Since the distal portion 12 of the guide wire 1 requires a flexibility, the elongation core 2 is thinned. For this reason, the guide wire 1 has the distal portion 12 liable to hang under the influence of gravity as the front helical spring tube 31 becomes heavy. This holds true when the distal portion 12 of the guide wire 1 navigates in the blood streams when inserted into the blood vessel.

(c) Upon inserting the guide wire 1 into the blood vessel, the distal portion 12 of the guide wire 1 generally hangs along the vascular wall, thus increasing a contact area with the vascular wall so as to invite a vascular rupture and a media rupture. Especially at bifurcated portions of the blood vessel, it becomes difficult to selectively manipulate which way to insert the distal portion 12 of the guide wire 1 at the bifurcated portions of the blood vessel.

(d) With the flotage chamber 5 provided in the distal portion 12 of the guide wire 1, it is possible to mitigate the distal portion 12 from hanging in the blood streams, thus making it possible to ride the distal portion 12 on the blood streams to smoothly navigate it deep into the sinuous and meandered blood vessel.

(e) With the flotage chamber 5 air-tightly sealed, it is possible to maintain a good elasticity of the flotage chamber 5 so as to maintain a good restitutive force appeared after manipulatively bending the distal portion 12 of the guide wire 1.

As a radiopaque material for the front helical spring tube 31, a metal such as gold, silver, tungsten may be selected.

As a radiotransparent material for the rear helical spring tube 32, a stainless steel may be preferably selected because of its good biocompatibility.

The radiotransparent material such as a platinum wire is liable to deform easily with a small amount of springback as compared to a stainless steel wire. Upon winding a linear wire (0.072 mm in diameter) into a helical spring (0.355 mm in outer diameter), it was found that the helical spring (made of the platinum wire) deforms diametrically smaller than the helical spring (made of the stainless steel wire) by 0.02 mm or more. Because the front helical spring tube 31 deforms easily, it is possible to provide a bending tendency with the front helical spring tube 31, and navigate the distal portion 12 of the guide wire 1 staunchly along the sinuous and meandered path upon inserting it deep into the blood vessel.

(f) With the flotage chamber 5 air-tightly sealed in the distal portion 12 of the guide wire 1, the deformation of the flotage chamber 5 increases its inside pressure, and an increased pressure tends to restitute the flotage chamber 5 after released from the deformation.

With the use of the elastic restitution of the flotage chamber 5, it is possible to add an tendency to the distal portion 12 to favorably hold its initial configuration.

Due to the difference of springback between the front helical spring tube 31 and the rear helical spring tube 32, it is possible to diametrically reduce the distal portion 12 of the guide wire 1 progressively as approaching forward.

The flotage chamber 5 effectuates a shape-holding advantage for the distal portion 12 of the guide wire 1 to significantly improve its passage against the stenotic area of the blood vessel.

(g) With the distal portion 12 riding on the blood streams to navigate deep into a somatic body, it becomes possible to thin the distal portion 12 of the guide wire, thus making it less intrusive to mitigate the burden the patient owes.

By way of illustration, upon implementing the therapeutic dilatation against the cardiovascular stenosis area, i.e., percutaneous transluminal coronary angioplasty (PTCA), a guide wire (0.35 mm in outer diameter) and a catheter (7F-8F: 2.3-2.7 mm in inner diameter) are used to introduce a balloon to dilate the cardiovascular stenosis area. The guide wire used for the therapeutical manipulation is generally 0.355 mm in outer diameter.

With the multi-stepped flat portion 27 defined on the elongation core 2, it is possible to insure a larger spatial area for the flotage chamber 5 between the elongation core 2 and the helical spring 3 as evidenced below.

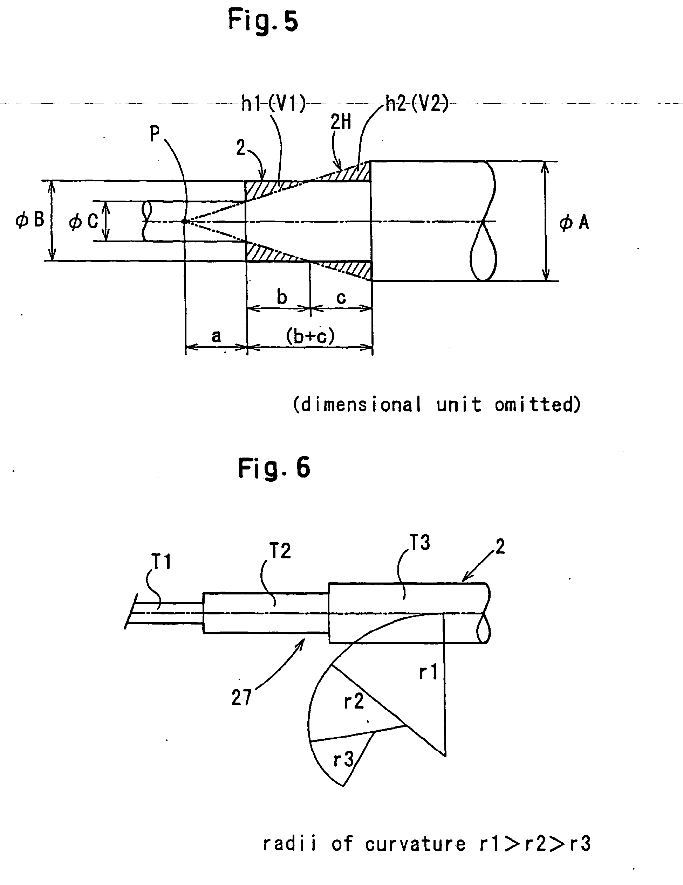

As shown in Fig. 5, a taper-shaped core 2H is adopted to compare it to the multi-stepped flat core 2 with the common latitudinal cross sectional area secured between the former and the latter. Hatched portions h1, h2 (triangular in shape) are depicted by dividing sections at an intersection between a tapered surface line and a horizontal line. Volumous difference between the two cores 2H, 2 equal to an arithmetical difference between annular volumes V2, V1. The volume V2 is derived by turning the hatched portion h2 around a central shaft S, and the volume V1 is derived by turning the hatched portion h1 around the central shaft S.

The volumes V2, V1 are calculated by using formulas involving a cylinder body and a frustocone-shaped body based on the dimensions (designated by denotations a, b, c, A, B, C). A schematic drawing in Fig. 5 represents an initial point (P) of the dimension (a) as a original point of the coordinates with no dimensional unit denoted.

$$V1 = \{ (B/2)^2 \times b \}\, \pi - \{ (1/3) \times (B/2)^2 \times (a+b) -$$

$$(1/3) \times (C/2)^2 \times a \}\, \pi$$

$$= \{ (B/2)^2 \times b - (1/3) \times (B/2)^2 \times (a+c) + (1/3) \times (C/2)^2 \times a \}\, \pi$$

$$V2 = \{ (A/2)^2 \times (1/3) \times (a+b+c) - (B/2)^2 \times (a+b) \times (1/3) - (B/2)^2 \times c \}\, \pi$$

Calculating the volumous difference V2-V1, V2-V1 = {- $(B/2)^2 \times (b+c) + (A/2)^2 \times (1/3) \times (a+b+c) - (C/2)^2 \times (1/3) \times a$} $\pi$ is obtained.

Considering the geometrical relationship in Fig. 5, formulas of a/C = (a+b)/B = (a+b+c)/A = m (constant) are obtained. From these formulas, (a+b) = Bm, (a+b+c) = Am, (b+c) = (A-C)m, c = (A-B)m are expressed.

With these expressions in mind, the following formulas are achieved.

$$V2 = \{ (A/2)^2 \times (A/3) - (B/2)^2 \times (B/3) - (B/2)^2 \times (A-B) \}\, m\, \pi$$

$$= \{ A^3 \times (1/12) - B^3 \times (1/12) - B^2 \times (A-B) \times (1/4) \}\, m\, \pi$$

$$= \{ (A-B) \times (A^2 + AB - 2B^2) \times (1/12) \}\, m\, \pi$$

**EP 1 767 239 B1**

$$V2-V1 = \{ -(B/2)^2 \times (A-C)+(A/2)^2 \times (A/3)-(C/2)^2 \times (C/3) \} \, m\pi$$

$$= \{ -B^2 \times (A-C) \times (1/4)+A^3 \times (1/12)-C^3 \times (1/12) \} \, m\pi$$

$$= [ \{ A^3-C^3-3B^2 \times (A-C) \} \times (1/12)]m\pi$$

$$= \{ (A-C) \times (A^2+ C^2+ AC-3B^2) \times (1/12) \} \, m\pi$$

Based on the volumes V1, v2, a percentage expression of $(V2-V1) \times 100/V2 = \{(A-C) \times (A^2 + C^2+ AC- 3B^2)\}$ / $\{(A-B) \times (A^2+AB-2B^2)\} \times 100$ % is obtaind.

In this situation, a diameter-enlarged section and a diameter-reduced section of the core 2H are in turn (A) mm and (C) mm, while an outer diameter of the elongation core 2 is (B) mm in a cylindrical shape. It is supposed that the pressing procedure remains the volume of the elongation core 2 substantially constant.

The percentage based on the volumes V1, V2 depends on the diametrical dimensions (A > B > C) regardless of the dimensions (a, b, c). By tangibly predetermining the! dimensions (A, B, C), the percentage based on the volumes V1, V2 can be calculated as desired. Especially in in view of the percentage formula, by predetermining a relative ratio of the dimension (A) against the dimensions (B, C) to be greater, it is possible to insure a much larger spatial area for the flotage chamber 5 between the elongation core 2 and the helical spring 3, comparing to tapered elongation core 2H can achieves.

Due to the multi-stepped flat portion 27, as shown in Fig'. 6, it is possible to bend stepped sections T1, T2 and T3 of the multi-stepped flat portion 27 at different radii of curvature r1, r2 and r3, thus enabling the manipulator to readily bend the distal end portion 21 of the elongation core 2 and insert it deep into a stenotic area staunchly along a sinuously formed path in the blood vessel.

Because of a buoyance derived from the flotage chamber 5, it is possible to make the distal end portion 21 ride on the blood streams to make it insert deep into the blood vessel. For this reason, the buoyance mitigates the mechanical requirements (e.g., torque transmissibility) for the guide wire 1 to permit the elongation core 2 to be thinned.

By way of illustration, the buoyance enables manufacturers to reduce the guide wire 1 from 0.014 to 0.010 inches (0.356 to 0.254 mm) in outer diameter, while at the same time, thinning a catheter from 7F-8F (2.3-2.7 mm in inner diameter) to 5F-6F (1.7-2.0 mm in inner diameter).

With the helical spring 3 formed from the front helical spring tube 31 and the rear helical spring tube 32, and the flotage chamber 5 provided in the front helical spring tube 31, it is possible to ameliorate an entire balance and prevents the distal portion 12 from being hung heavily.

In the guide wire 1 used for the percutaneous transluminal coronary angioplasty (PTCA), the elongation core 2 is tapered from the rear helical spring tube 32 to a distal end of the front helical spring tube 31. Since the specific gravity of the front helical spring tube 31 is greater with the distal end portion 21 thinned, the guide wire 1 has the distal portion 12 liable to hang as approaching forward.

With the flotage chamber 5 provided in the distal portion 12 of the guide wire 1, it is possible to effectively mitigate the distal portion 12 from being hung in the blood streams, thus making it possible to substantially maintain it straight so as to avoid a vascular rupture and a media rupture in the blood vessel.

The flotage chamber 5 is air-tightly sealed positively by the brazing portion 10, the hermetical wall 11 and the synthetic coat 4. The distal end portions of the elongation core 2 and the helical spring 3 are tightly fixed by means of a brazing procedure (tin pellets), brazing procedure or the like. A proximal end of the front helical spring tube 31 is tightly fixed to the elongation core 2 by means of a brazing procedure (tin pellets), brazing procedure or the like.

Thereafter, the synthetic coat 4 are applied to an outer surface of the front helical spring tube 31 to at least cover an entire surface of the flotage chamber 5.

It is to be noted that the synthetic coat 4 may be applied to a whole part of the helical spring 3 and the elongation core 2. As for a method of forming the synthetic coat 4, an extrusion procedure, a dipping procedure or a thermal shrinkage tube procedure may be used so long as the method is effective in air-tightly sealing the flotage chamber 5.

Among the methods raised above, the dipping procedure and the thermal shrinkage tube procedure are preferable since they prevents the synthetic coat 4 from invading into the flotage chamber 5 with the gaseous component left intact inside the flotage chamber 5. The dipping procedure is also preferable since it neither needs to pressurize the flotage chamber 5 nor needs to thermally deal with the ends of the synthetic coat 4. In order to avoid the gaseous leakage from the flotage chamber 5, double layers of the synthetic coat may be provided.

It is also to be noted that a viscous fluid layer 42 (different in viscosity from the blood streams) may be provided as a hydrophilic polymer on an outermost surface of the double layers. The fluid layuer 42 serves as a second lubricating layer to exhibit a lubricity when moistened.

With the outer surface of the helical spring 3 covered by the synthetic coat 4, it is possible to maintain a good elasticity of the flotage chamber 5 so as to hold a good restitutive force appeared after manipulatively bending the distal portion 12 of the guide wire 1, while at the same time, protecting the elongation core 2 against the plastic deformation.

Due to the double layers forming the viscous fluid layer 42 on the first solid layer (e.g., polyurethane layer) of the synthetic coat 4, the following advantages are obtained.

Even if minute pores (pinholes) or injuries are developed on the synthetic coat 4, it is possible to avoid the gaseous leakage of the flotage chamber 5 so as to air-tightly maintain the flotage chamber 5 by covering an entire surface of the synthetic coat 4 with the viscous fluid layers 42.

By covering the.synthetic coat 4 with the viscous fluid layer 42, it is possible to mitigate the friction of the synthetic coat 4 against the vascular wall in the blood vessel.

Upon forming the synthetic coat 4 from a mixture of a hydrophilic polymer and a hydrophobic polymer, it is possible to provide the synthetic coat 4 with a first layer which contains the hydrophobic polymer more than the hydrophilic polymer by weight, and having an outer layer which contains the hydrophilic polymer more than the first layer contains the hydrophilic polymer by weight. The hydrophilic polymer of the outer layer increases progressively by weight as approaching an outer surface of the'outer layer. This makes it possible to more air-tightly seal the flotage chamber 5, while and at the same time, mitigating the friction of the synthetic coat 4 against the vascular wall in the blood vessel.

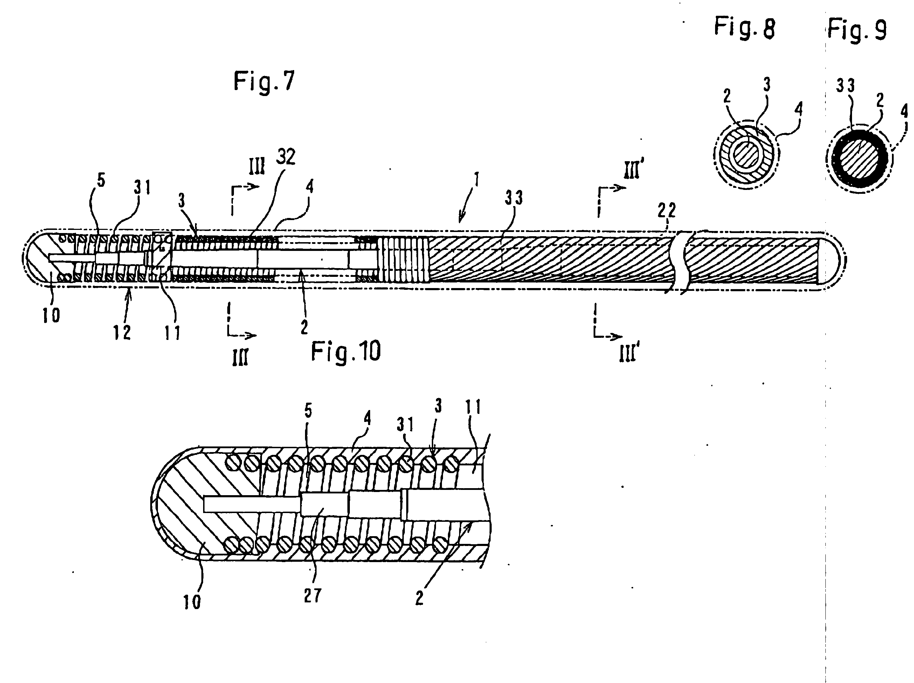

Figs. 7 through 10 show a second embodiment of the invention in which a multi-stranded helical spring tube 33 is connected to a proximal side of the rear helical spring tube 32. Both rear ends of the elongation core 2 and the multi-stranded helical spring tube 33 are fixed by means of a welding or brazing procedure.

When comparing a solid core to the case in which the solid core is inserted to the multi-stranded helical spring under the common diametrical dimension at the proximal end portion, the multi-stranded helical spring tube develops a concave-shaped clearance between the neighboring helical line elements. This makes it possible to produce the lightweight elongation core as opposed to a'solid elongation core.

Upon inserting the elongation core 2 into the blood vessel, the blood streams run along the helical line elements of the multi-stranded helical spring tube 33 to give the elongation core 2 a propelling force, thus enabling the manipulator to navigate it deep into the stenotic area of the blood vessel.

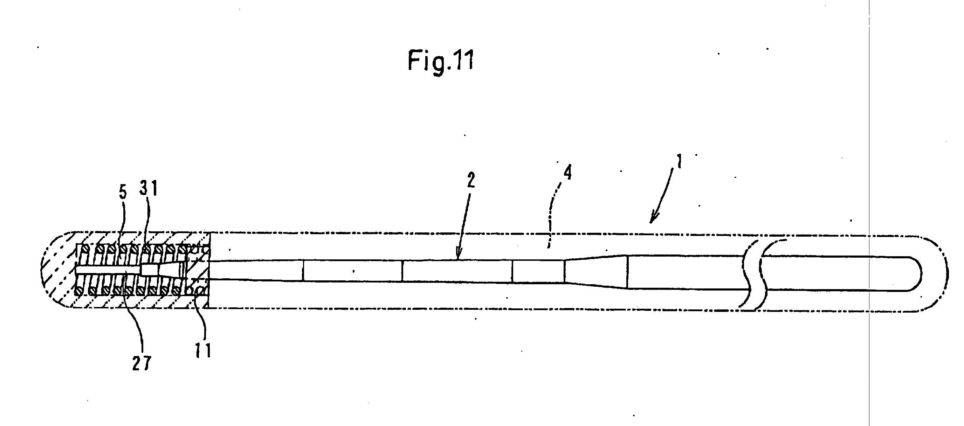

Fig. 11 shows a third embodiment of the invention in which the front helical spring tube 31 forms all the helical spring 3. In this instance, an entire surface of the front helical spring tube 31 is covered by the synthetic coat 4 to provide the guide wire 1.

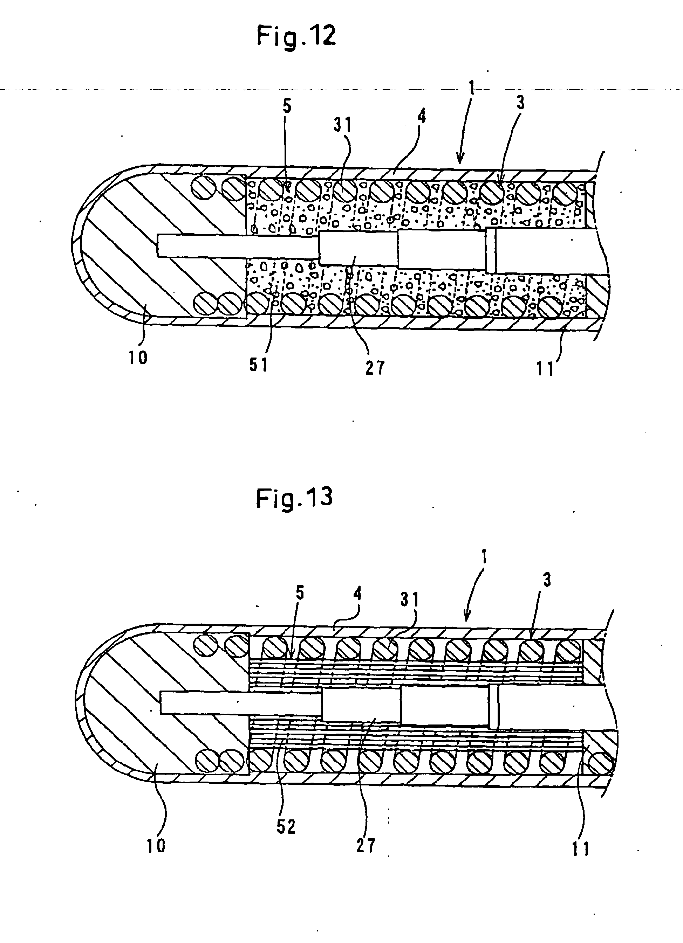

Fig. 12 shows a fourth embodiment of the invention in which the flotage chamber 5 is formed by filling a foamy substance (sponge) 51 between the helical spring 3 and the elongation core 2. In this instance, the foamy substance 51 is provided by dipping the distal end portion 21 of the elongation core 2 into a foamy liquid bath after brazing the helical spring 3 to the elongation core 2. Then, the elongation core 2 is withdrawn from the foamy liquid bath, and trimmed at the foamy substance 51 to be diametrically constant in the lengthwise direction with the use of a jig tool.

Thereafter, the foamy substance 51 is heated or left as it is until solidified. With the use of the dipping procedure, the synthetic coat 4 is covered with an entire surface of the helical spring 3 and the elongation core 2. It is to be noted that a spray may be used upon providing the foamy substance 51.

The foamy substance 51 is provided by adding a foamy agent to a synthetic resin. The synthetic resin represents polyester, copolymer of styrene and methacrylic acid (styrene-based resin) and polyethylene, polypropylene (polyolefin-based resin). The foaming agent represents carbon dioxide (volatile) and ammonium carbonate (dissoluble). By way of example, a bridged bond polyolefin foaming agent (specific gravity: 0.06-0.3) may be used.

The foamy substance 51 may be formed by adding the foamy agent to a rubber (silicone rubber, chloroprene rubber). The foamy agent for the silicone rubber represents azobisisobutylnitrile. As for the foamy substance 51, a texture in which foams are discretely arranged is preferable to a texture in which foams are continuously arranged. The minute foams contained in the foamy substance 51 are preferable. A silicone sponge (minute cell-texture and 110 $\mu$m on average cellular diameter) is preferable which has a low density relative to water (approx. 0.41) with an excellent permanent strain exhibited when a contractile stress is applied.

With the foamy substance 51 placed between the elongation core 2 and the helical spring 3, it is possible to preven the synthetic from invading into the flotage chamber 5 even if the helical spring 3 is depressed upon applying the synthetic coat 4 to the outer surface of the helical spring 3 by means of an extrusion procedure.

With the foamy substance 51 formed by the discretely arranged-foam texture, it is possible to effectively avoid the synthetic resin from invading into the flotage chamber 5 upon applying the synthetic coat 4 to the outer surface of the helical spring 3.

Due to the foamy substance 51 being of an elastic material, it effectively prevents the elongation core 2 and the helical spring 3 from being plastically deformed, so as to insure an increased restitutive force appeared after the distal end of the guide wire 1 is manipulatively bent.

In order to provide the flexibility with the front helical spring tube 31, it is arranged to develop a tiny clearance between the helical line elements of the front helical spring tube 31. If the synthetic resin invades into the tiny clearance between the helical line elements upon forming the synthetic coat 4, it would hinder the good flexibility of the front helical spring tube 31.

With the flotage chamber 5 formed by the foamy substance 51, the foamy substance 51 extends over the outer surface of the front helical spring tube 31, thus maintaining the good flexibility of the front helical spring tube 31.

Fig. 13 shows a fifth embodiment of the invention in which the flotage chamber 5 is formed with cotton fibers or a bundle of fibers 52. They represents polyethylene fibers, para-aramid fibers and PBO fibers. It is preferable that shapes of the fibers (hollow fibers) are such as to contain a gaseous component when the fibers are bundled. The fibers (2-100 $\mu$m in thickness) may be bundled in a braid-like configuration. Biocompatible fibers may be used as bioabsorbable polymer fibers (e.g., biodegradable polylactic acid). The fibers measure 0.5-50 $\mu$m in diameter and 3-50 mm in length.

With the use of of very thin fibers (2-10 $\mu$m), it is possible to form the flotage chamber 5 with the bundle of the fibers 52 which contain a greater amount of the gaseous component. The bundle the fibers 52 favorably maintains the flexibility required for the distal end 12 of the guide wire 1. By appropriately winding the braid around the elongation core 2, it is possible to adjust a wound length of the braid so as to readily form the flotage chamber 5. Upon using the bioabsorbable polymer fibers, it is possible to decompose the fibers within the body, thus involving no complication disorder with no uncomfortable feeling given to the patient even if the fibers flow into the blood streams.

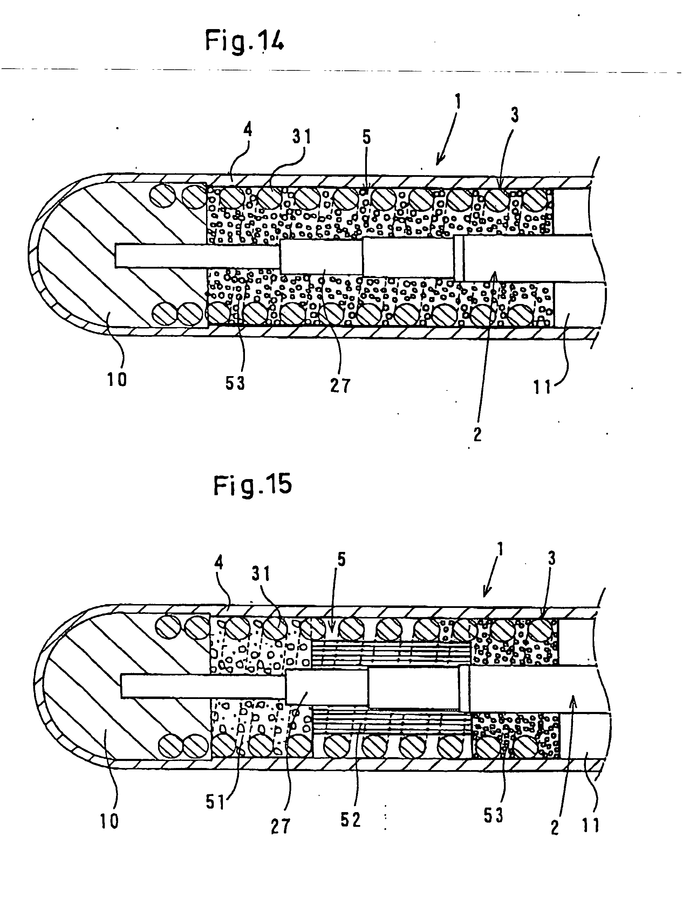

Fig. 14 shows a sixth embodiment of the invention in which the flotage chamber is formed with globular grains 53 (synthetic foamy beads, microballoons). The material for the globular grains 53 (e.g., 0.06-0.5 and 50-100 $\mu$m in terms of density relative to water and granular size) is selected from the chemical components raised in the fourth embodiment of the invention.

As the microballoons, minute and inorganic globular grains (e.g., vitreous, alumina or silica) are selected (e.g., 0.2-0.7 and 1-150 $\mu$m in terms of the specific gravity and granular size). The flotage chamber 5 may be formed with a single one substance selected from the synthetic foamy beads and the microballoons. A mixture (binder) of the rubber and the synthetics, the foamy substance 51 or the bundle:of fibers 52 may be used upon forming the flotage chamber 5. The globular grains 53 may be formed by the microballoons (e.g., 0.2 and 10 $\mu$m in terms of density relative to water and granular size) mixed with the foamy substance 51.

With the foamy beads or microballoons having less chances to come in contact with the neighboring beads or microballoons, it is possible to insure larger spatial portions favorable to provide the flotage chamber 5. With the use of inorganic microballoons, it is possible to increase the contractile strength for the distal end portion 12 of the guide wire 1 when manipulatively bent without leaking the gaseous component from the flotage chamber 5. It is preferable that lightweight gas (e.g., helium) may be contained in the flotage chamber 5 to increase the buoyance for the flotage chamber 5.

By using the foamy substance 51 as a binder for the globular grains 53, it is possible to readily form the flotage chamber 5 within the front helical spring tube 31, while at the same time, increasing the buoyance by containing the lightweight gas in the flotage chamber 5.

Upon forming the flotage chamber 5, the same method can be used as mentioned in the fourth embodiment of the invention except for the process in which a certain amount of the globular grains 53 is added to the foamy substance 51.

Fig. 15 shows a seventh embodiment of the invention in which the flotage chamber 5 is formed by the globular grains 53, the foamy substance 51, the bundle of fibers 53 or a compound body of these substances. The proximal end of the helical spring 3 may be brazed to the elongation core 2.

In a prior medical guide wire, a radiopaque helical spring is tightly secured to an elongation core by means of a caulking procedure or an adhesive in order to avoid positional displacements between the elongation core and the radiopaque helical spring. This reduces the flexibility required for a distal end of the guide wire.

As opposed to the prior art counterpart, the elongation core 2 and the helical spring 3 are fixed by means of the globular grains 53, the foamy substance 51 or the bundle of fibers 53 in the subject guide wire 1.

For this reason, the helical spring 3 tightly pushes its inner undulating surface against the globular grains 53, thus forming the synthetic coat 4 without inviting the positional displacements between the elongation core 2 and the front helical spring tube 31. The formation of synthetic coat 4 prevents the distal end of the guide wire 1 from losing its flexibility, and effectively avoiding the' elongation core 2 and the helical spring 3 from being plastically deformed, so as to insure an increased restitutive force appeared after the distal end of the guide wire 1 is manipulatively bent.

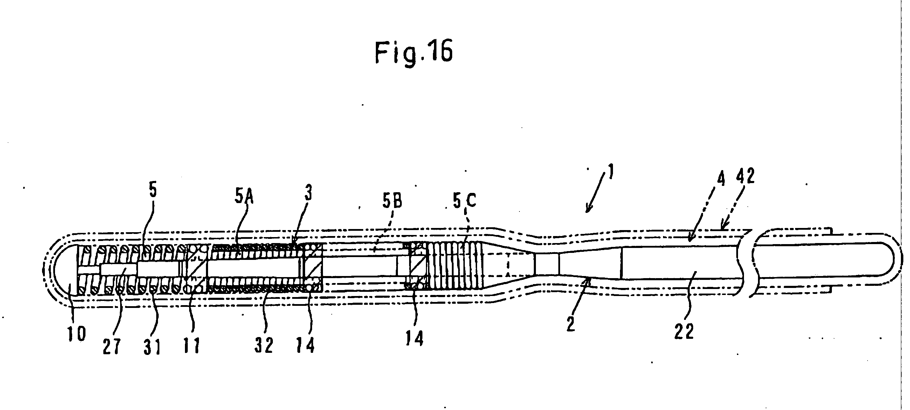

Fig. 16 shows an eighth embodiment of the invention in' which a flotage chamber 5A is formed behind the hermetical wall 11 in addition to the flotage chamber 5 provided inside the front helical spring tube 31.

In this instance, hermetical walls 14 are provided inside the rear helical spring tube 32 at regular intervals, and flotage chambers 5A, 5B, 5C are formed inside the rear helical spring tube 32 so as to increase the buoyance for the distal end 12 of the guide wire 1.

The synthetic coat 4 extends from the distal end to the proximal end of the helical spring 3 to air-tightly cover the entire surface of the helical spring 3. The elongation core 2 and the helical spring 3 are fixed by means of not only the brazing procedure but also the plasma welding procedure or the TIG welding procedure so long as the procedures maintain the hermetic seal for the flotage chambers. It is to be noted that the synthetic coat 4 may cover most part of the elongation core 2 as shown in Fig. 16.

In this situation, it is possible to make the buoyance increase progressively as approaching forward, while at the same time, making the buoyance adjustable by selecting ones among the flotage chambers 5A, 5B, 5C. This enables the manipulator to favorably direct the distal end 12 of the guide wire 1 along the blood vessel so as to significantly improve a natatorial capability for the guide wire 1.

**Claims**

1. A medical guide wire (1) in which a helical spring (3) is provided to have a radiopaque portion and a radiotransparent portion, each of which is made of a radiopaque material and a radiotransparent material respectively, said radiopaque portion is defined at a distal end portion of said helical spring (3), and said radiotransparent portion is defined at a proximal end portion of said helical spring (3), an elongate core (2) having a thinned portion at a distal end portion (21) and a thickened portion at a proximal end portion (22) of said elongate core (2), said distal end portion (21) of said elongate core (2) being inserted to be placed within said radiopaque portion of said helical spring (3), both the distal end portions of said helical spring (3) and said elongate core (2) being air-tightly fixed with an outer surface of said helical spring (3) being covered by a synthetic coat (4);
a hermetical wall (11) is provided to air-tightly fix said helical spring (3) to said elongate core (2) at a proximal end portion of said radiopaque portion of said helical spring (3),
a flotage chamber (5) is formed within said radiopaque portion of said helical spring (3) as an inner space surrounded by a fixing portion (10), said hermetical wall (11) and said synthetic coat (4), said fixing portion (10) being formed by said helical spring (3) and said elongate core (2),
a deformation of said flotage chamber (5) increases an inside pressure of said flotage chamber (5) at the time of bending said radiopaque portion of said helical spring (3), and an increased inside pressure restitutes said flotage chamber (5) at the time of releasing said flotage chamber (5) from said formation; **characterised in that** said helical spring (3) is provided by a radiopaque material and a radiotransparent material connected by means of a fixing procedure, and extruded to be diametrically reduced, and wound so as to define a radiopaque helical spring at the distal end portion of said helical spring (3), wherein a distal end portion of said helical spring (3) has a radiopaque portion formed by a radiopaque material, an amount of spring-back of which is smaller than that of a stainless steel metal, and the distal portion of the guide wire is progessively diametrically reduced as approaching forward.

2. A medical guide wire (1) according to claim 1,
wherein the thickness of the distal end portion (21) stepwisely decreases as approaching said distal end of said elongate core (2) so as to form a multi-stepped flat portion (27) at a distal end portion (21) of said elongate core (2).

3. The medical guide wire (1) according to claim 1 or 2, wherein said synthetic coat (4) includes a solid layer as a first hydrophobic layer and a fluid layer (42) serving as a second lubricating layer to exhibit a lubricity when moistened.

4. The medical guide wire (1) according to claim 1 or 2, wherein said synthetic coat is a mixture of a hydrophilic polymer and a hydrophobic polymer, and having a first layer which contains said hydrophobic polymer more than said hydrophilic polymer in weight, and having an outer layer which contains said hydrophilic polymer more than said first layer contains said hydrophilic polymer in weight, said hydrophilic polymer of said outer layer increasing progressively in weight as approaching an outer surface of said outer layer.

5. The medical guide wire (1) according to any preceding claim, wherein a foamy body is placed in said flotage chamber formed between said helical spring (3) and said elongate core (2).

6. The medical guide wire (1) according to any of claims 1 to 5, wherein foamy beads or microballoons are encapsulated in said flotage chamber (5) formed between said helical spring (3) and said elongate core (2) by means of a fixing procedure.

7. The combination of a catheter and said medical guide wire (1) according to any of claims 1 to 6, wherein an outer diameter of said medical guide wire (1) is 0.2541 mm (0.01 inches) and said medical guide wire is adapted to be inserted into said catheter, an inner diameter of which ranges from 1.7 mm to 2.0 mm.

**Patentansprüche**

1.  Medizinischer Führungsdraht (1), in dem eine Schraubenfeder (3) vorgesehen ist, die einen strahlenundurchlässigen Abschnitt und einen strahlendurchlässigen Abschnitt aufweist, wobei jeder derselben aus einem strahlenundurchlässigen Material bzw. einem strahlendurchlässigen Material besteht, der strahlenundurchlässige Abschnitt an einem distalen Endabschnitt der Schraubenfeder (3) ausgebildet ist und der strahlendurchlässige Abschnitt an einem proximalen Endabschnitt der Schraubenfeder (3) ausgebildet ist, wobei ein länglicher Kern (2) einen verdünnten Abschnitt an einem distalen Endabschnitt (21) und einen verdickten Abschnitt an einem proximalen Endabschnitt (22) des länglichen Kerns (2) aufweist, wobei der distale Endabschnitt (21) des länglichen Kerns (2) so eingesetzt ist, dass er in dem strahlenundurchlässigen Abschnitt der Schraubenfeder (3) positioniert ist, wobei die distalen Endabschnitte der Schraubenfeder (3) und des länglichen Kern (2) beide durch Abdecken mit einem Kunststoffmantel luftdicht mit einer Außenfläche der Schraubenfeder (3) befestigt sind;
    eine hermetische Wand (11) vorgesehen ist, um die Schraubenfeder (3) luftdicht an dem länglichen Kern (2) an einem proximalen Endabschnitt des strahlenundurchlässigen Abschnitts der Schraubenfeder (3) zu befestigen,
    in dem strahlenundurchlässigen Abschnitt der Schraubenfeder (3) eine Schwimmkammer (5) als Innenraum ausgebildet ist, der durch einen Befestigungsabschnitt (10), die hermetische Wand (11) und den Kunststoffmantel (4) umgeben ist, wobei der Befestigungsabschnitt (10) durch die Schraubenfeder (3) und den länglichen Kern (2) ausgebildet ist,
    eine Verformung der Schwimmkammer (5) zum Zeitpunkt des Biegens des strahlenundurchlässigen Abschnitts der Schraubenfeder (3) einen Innendruck der Schwimmkammer (5) erhöht und ein erhöhter Innendruck zum Zeitpunkt des Lösens der Schwimmkammer (5) aus der Formung die Schwimmkammer (5) wieder herstellt;
    **dadurch gekennzeichnet, dass** die Schraubenfeder (3) durch ein strahlenundurchlässiges Material und ein strahlendurchlässiges Material vorgesehen ist, die mittels eines Befestigungsverfahrens verbunden sind und so stranggepresst sind, dass im Durchmesser verringert sind, und so gewunden sind, dass sie eine strahlenundurchlässige Schraubenfeder (3) am distalen Endabschnitt der Schraubenfeder (3) ausbilden, wobei ein distaler Endabschnitt der Schraubenfeder (3) einen durch ein strahlenundurchlässiges Material ausgebildeten strahlenundurchlässigen Abschnitt aufweist, wobei ein Rückfederungsbetrag desselben kleiner als der eines Edelstahlmetalls ist, und der distale Abschnitt des Führungsdrahts nach vorne hin zunehmend im Durchmesser abnimmt.

2.  Medizinischer Führungsdraht (1) nach Anspruch 1, **dadurch gekennzeichnet, dass** die Dicke des distalen Endabschnitts (21) zum distalen Ende des länglichen Kerns (2) hin stufenweise abnimmt, um so einen mehrstufigen flachen Abschnitt (27) an einem distalen Endabschnitt (21) des länglichen Kerns (2) auszubilden.

3.  Medizinischer Führungsdraht (1) nach Anspruch 1 oder 2, **dadurch gekennzeichnet, dass** der Kunststoffmantel (4) eine feste Schicht als erste hydrophobe Schicht und eine Fluidschicht (42) umfasst, die als zweite Schmierschicht dient, um bei Befeuchten Schmierfähigkeit aufzuweisen.

4.  Medizinischer Führungsdraht (1) nach Anspruch 1 oder 2, **dadurch gekennzeichnet, dass** der Kunststoffmantel eine Mischung aus einem hydrophilen Polymer und einem hydrophoben Polymer ist und eine erste Schicht aufweist, die gewichtsmäßig mehr hydrophobes Polymer als hydrophiles Polymer enthält, und eine Außenschicht aufweist, die gewichtsmäßig mehr hydrophiles Polymer enthält, als die erste Schicht hydrophiles Polymer enthält, wobei das hydrophile Polymer der Außenschicht zu einer Außenfläche der Außenschicht hin zunehmend an Gewicht gewinnt.

5.  Medizinischer Führungsdraht (1) nach einem der vorhergehenden Ansprüche, **dadurch gekennzeichnet, dass** in der zwischen der Schraubenfeder (3) und dem länglichen Kern (2) ausgebildeten Schwimmkammer ein Schaumstoffkörper positioniert ist.

6.  Medizinischer Führungsdraht (1) nach einem der Ansprüche 1 bis 5, **dadurch gekennzeichnet, dass** Schaumstoffkugeln oder Mikro-Ballons in der zwischen der Schraubenfeder (3) und dem länglichen Kern (2) ausgebildeten Schwimmkammer (5) mittels eines Befestigungsverfahrens eingekapselt sind.

7.  Kombination aus einem Katheter und dem medizinischen Führungsdraht (1) nach einem der Ansprüche 1 bis 6, **dadurch gekennzeichnet, dass** ein Außendurchmesser des medizinischen Führungsdrahts (1) 0,2541 mm (0,01 Zoll) beträgt und der medizinische Führungsdraht dafür ausgelegt ist, in den Katheter eingesetzt zu werden, dessen Innendurchmesser 1,7 mm bis 2,0 mm beträgt.

**Revendications**

1.  Fil de guidage médical (1), dans lequel un ressort hélicoïdal (3) est disposé de façon à comporter une partie radio-opaque et une partie radio-transparente, chacune d'entre elles étant constituée par un matériau radio-opaque et un matériau radio-transparent, respectivement, ladite partie radio-opaque étant définie à une partie d'extrémité distale dudit ressort hélicoïdal (3), et ladite partie radio-transparente étant définie à une partie d'extrémité proximale dudit ressort hélicoïdal (3), un coeur allongé (2) comportant une partie amincie à une partie d'extrémité distale (21) et une partie épaissie à une partie d'extrémité proximale (22) dudit coeur allongé (2), ladite partie d'extrémité distale (22) dudit coeur allongé (2) étant insérée de façon à être disposée à l'intérieur de ladite partie radio-opaque dudit ressort hélicoïdal (3), les deux parties d'extrémité distale dudit ressort hélicoïdal (3) et dudit coeur allongé (2) étant fixées de façon étanche vis-à-vis de l'air à une surface extérieure dudit ressort hélicoïdal (3) recouverte par un revêtement synthétique (4) ;
    une paroi hermétique (11) étant disposée de façon à fixer de manière étanche vis-à-vis de l'air ledit ressort hélicoïdal (3) audit coeur allongé (2) à une partie d'extrémité proximale de ladite partie radio-opaque dudit ressort hélicoïdal (3), une chambre de flottaison (5) étant formée à l'intérieur de ladite partie radio-opaque dudit ressort hélicoïdal (3) sous la forme d'un espace intérieur entouré par une partie de fixation (10), ladite paroi hermétique (11) et ledit revêtement synthétique (4), ladite partie de fixation (10) étant formée par ledit ressort hélicoïdal (3) et ledit coeur allongé (2), une déformation de ladite chambre de flottaison (5) accroissant une pression intérieure de ladite chambre de flottaison (5) au moment d'une courbure de ladite partie radio-opaque dudit ressort hélicoïdal (3), et une pression intérieure accrue rétablissant ladite chambre de flottaison au moment d'un relâchement de ladite chambre de flottaison (5) à partir de ladite formation ; **caractérisé en ce que :**

    ledit ressort hélicoïdal (3) est constitué par un matériau radio-opaque et un matériau radio-transparent reliés à l'aide d'une procédure de fixation, et extrudé de façon à être diamétralement réduit, et enroulé de façon à définir un ressort hélicoïdal radio-opaque à la partie d'extrémité distale dudit ressort hélicoïdal (3), une partie d'extrémité distale dudit ressort hélicoïdal (3) comportant une partie radio-opaque formée par un matériau radio-opaque, dont une ampleur de retour élastique est inférieure à celle d'un métal en acier inoxydable, et la partie distale du fil de guidage étant progressivement réduite de façon diamétrale lorsqu'elle se rapproche de l'avant.

2.  Fil de guidage médical (1) selon la revendication 1, dans lequel l'épaisseur de la partie d'extrémité distale (21) diminue par pas lorsqu'elle s'approche de ladite extrémité distale dudit coeur allongé (2) de façon à former une partie plate à pas multiples (27) à une partie d'extrémité distale (21) dudit coeur allongé (2).

3.  Fil de guidage médical (1) selon la revendication 1 ou 2, dans lequel ledit revêtement synthétique (4) comprend une couche solide constituant une première couche hydrophobe et une couche fluide (42) constituant une deuxième couche lubrifiante afin de présenter une capacité de lubrification lorsqu'elle est humidifiée.

4.  Fil de guidage médical (1) selon la revendication 1 ou 2, dans lequel ledit revêtement synthétique est un mélange d'un polymère hydrophile et d'un polymère hydrophobe, et comportant une première couche qui contient ledit polymère hydrophobe en plus grande quantité en poids que ledit polymère hydrophile, et comportant une couche extérieure qui contient ledit polymère hydrophile en plus grande quantité en poids que ladite première couche ne contient ledit polymère hydrophile, le poids dudit polymère hydrophile de ladite couche extérieure augmentant progressivement lorsqu'il s'approche d'une surface extérieure de ladite couche extérieure.

5.  Fil de guidage médical (1) selon l'une quelconque des revendications précédentes, dans lequel un corps en mousse est disposé dans ladite chambre de flottaison formée entre ledit ressort hélicoïdal (3) et ledit coeur allongé (2).

6.  Fil de guidage médical (1) selon l'une quelconque des revendications 1 à 5, dans lequel des perles de mousse ou des micro-ballons sont encapsulés dans ladite chambre de flottaison (5) formée entre ledit ressort hélicoïdal (3) et ledit coeur allongé (2) à l'aide d'une procédure de fixation.

7.  Combinaison d'un cathéter et dudit fil de guidage médical (1) selon l'une quelconque des revendications 1 à 6, dans laquelle un diamètre extérieur dudit fil de guidage médical (1) est de 0,2541 mm (0,01 pouce) et ledit fil de guidage médical est adapté pour être inséré dans ledit cathéter, dont un diamètre intérieur est compris entre 1,7 mm et 2,0 mm.

Fig.1

Fig. 2

Fig.3

Fig. 4

EP 1 767 239 B1

# Fig. 5

(dimensional unit omitted)

# Fig. 6

radii of curvature r1＞r2＞r3

Fig. 8   Fig. 9

Fig. 7

Fig. 10

EP 1 767 239 B1

Fig.11

## Fig.12

## Fig.13

Fig.14

Fig.15

Fig.16

EP 1 767 239 B1

**REFERENCES CITED IN THE DESCRIPTION**

**Patent documents cited in the description**

- JP 2000135289 A
- JP 4009162 A
- JP 2588582 A
- EP 1498152 A1
- EP 0982046 A1
- EP 0666086 A1